(19) Europäisches Patentamt
European Patent Office
Office européen des brevets

(11) **EP 3 817 005 A1**

(12) **EUROPEAN PATENT APPLICATION**

(43) Date of publication:
**05.05.2021 Bulletin 2021/18**

(51) Int Cl.:
**G16H 50/20** (2018.01)    **G16H 50/30** (2018.01)

(21) Application number: **20150274.7**

(22) Date of filing: **03.01.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.11.2019 US 201962929540 P**

(71) Applicant: **Koninklijke Philips N.V.
5656 AG Eindhoven (NL)**

(72) Inventors:
• **FENG, Ting
5656 AE Eindhoven (NL)**
• **CONROY, Bryan
5656 AE Eindhoven (NL)**
• **NOREN, David Paul
5656 AE Eindhoven (NL)**
• **MCFARLANE, Daniel Craig
5656 AE Eindhoven (NL)**
• **SHREYAS, Ravindranath
5656 AE Eindhoven (NL)**
• **SCHWAGER, Emma
5656 AE Eindhoven (NL)**

(74) Representative: **Steenbeek, Leonardus Johannes et al
Philips Intellectual Property & Standards
High Tech Campus 5
5656 AE Eindhoven (NL)**

(54) **SYSTEM FOR INFECTION DIAGNOSIS**

(57)    A system (400) for diagnosing pathogenic infection of a person, the system comprising a processor (420) configured for: receiving (110) a trigger comprising data indicative of a possible pathogenic infection; determining (120), using a risk classifier and medical information about the patient, a risk score for the patient comprising a likelihood that one or more body systems is infected; determining (130), using a likelihood classifier and the medical information, a likelihood score for the patient comprising an identification of one or more pathogens or pathogen categories that could be causing an infection; determining (140) a relevance score using a relevance classifier and the determined risk and likelihood scores, the relevance score comprising one or more clinical tests relevant to confirming or rejecting the possible pathogenic infection of the person; and reporting (150), via a user interface, the determined relevance score.

FIG. 1

**EP 3 817 005 A1**

## Description

### Field of the Disclosure

The present disclosure is directed generally to systems for diagnosing a potential pathogenic infection.

### Background

Hospital-acquired infections result in 100,000 deaths per year, and bacterial infections are becoming increasingly difficult to treat. Accordingly, appropriate pathogen surveillance must be applied to prevent the spread of multidrug resistant pathogen within or across healthcare systems. Early and accurate diagnoses of infections are therefore critical such that targeted treatments and interventions can be delivered early to improve patient health outcomes, and such that early and effective infectious disease management can be utilized to avoid the spread of infection.

The diagnosis of infection typically requires two components. First, a doctor confirms that the patient's infection-like symptoms are indeed caused by infection. This is typically done via clinical testing to identify which body system is being infected. Second, the doctor identifies the microorganism that causes the infection, again via clinical testing. Both components are important for determining the appropriate clinical management of the infected patient.

However, identifying the invading microorganism and the infected body system is not a trivial task. Diseases may cause similar symptoms and induce similar physiological changes of the affected body system, but the underlying disease physiology and the corresponding treatment will be different. In order to obtain an accurate diagnosis, a doctor typically orders a series of clinical tests to gather more information about the illness, and to rule out other possible disorders. As each clinical test result takes time to obtain, early diagnosis becomes difficult, especially when the appropriate tests are not ordered immediately.

For example, a person with a cough and difficulty breathing may have pneumonia, which is a lung infection, or may instead have asthma or heart failure which are not caused by infection. If the doctor suspects pneumonia, a chest x-ray will be ordered to examine the patient's lung. Signs of infiltrate, or consolidation, or cavitation of the lung shown in the chest x-ray indicate that the lung is infected. Alternatively, if the doctor suspects heart failure, an electrocardiogram (ECG) may be ordered. In the situation that the patient does have pneumonia, chest x-ray is the most relevant test for diagnosis; ECG on the other hand can be used to rule out heart failure, but it cannot be used to diagnose pneumonia. If the patient is diagnosed with lung infection via the chest x-ray, the next step is to identify the invading microorganism. Bacterial pneumonia, viral pneumonia, and fungal pneumonia all cause similar symptoms such as cough and difficulty in breathing. However, they differ in their corresponding treatment and management. Antibiotics are the go-to medicine for treating bacterial pneumonia but they do not have any effect in treating viral pneumonia or fungal pneumonia. Additionally, bacterial pneumonia and viral pneumonia are contagious therefore efforts will be made to prevent disease from spreading, such as by moving the infected patient to a private room. However, there is less concern for fungal pneumonia as it is not contagious from person to person. To distinguish between microorganisms, the doctor typically obtains samples from the patient for further examination. This could be a nasal swab for flu if viral pneumonia is suspected, or a sputum sample sent to the laboratory for culture and for an examination under the microscope, if bacterial pneumonia is suspected. Only through these tests can the microorganisms be identified and therapeutic decisions made. This multi-step process is both slow and time-consuming. As such, it allows further time for the infection to develop and/or spread. Early diagnosis becomes nearly impossible.

### Summary of the Disclosure

There is a continued need for systems that enable early diagnosis and treatment of infection using big data and a machine learning approach.

The present disclosure is directed to inventive systems for early diagnosis of a potential pathogenic infection. Diagnosing infection is not a straightforward task, especially if a patient has other underlying disease that causes similar symptoms, and/or when invading microorganisms are difficult to identify. Doctors typically order clinical tests to help diagnosis, but relevant tests may be not easy to determine, which causes delay to the diagnosis of infection. As a consequence, targeted treatment cannot be initiated timely, thereby resulting poor patient outcome.

Accordingly, various embodiments and implementations herein are directed to a system that determines a likelihood of an infection, along with various characteristics and tests for that infection, at an early stage. The automated system provides healthcare providers with the following information to support their decisions: 1) a risk score comprising one or more predefined body systems measuring the risk of each being infected; 2) a likelihood score comprising one or more predefined microorganisms measuring the likelihood of each of them being the cause of infection; and 3) a relevance score comprising one or more predefined clinical tests measuring the relevance of each of them in infection diagnosis. According to an embodiment the scores are learned from retrospective clinical data using a hierarchical machine learning framework. Healthcare professionals can use this information to order relevant clinical tests, to enable early diagnosis, and to manage intervention.

Generally in one aspect, the invention provides a system for diagnosing pathogenic infection of a person. The system includes a processor for: (i) receiving a trigger at an infection analysis system, the trigger comprising data indicative of a possible pathogenic infection of the person; (ii) determining, using a risk classifier of the system and medical information about the patient (e.g. from one or more medical records for the patient), a risk score for the patient comprising a likelihood that one or more body systems is infected; (iii) determining, using a likelihood classifier of the system and medical information about the patient, a likelihood score for the patient comprising an identification of one or more pathogens or pathogen categories that could be causing an infection; (iv) determining a relevance score using a relevance classifier of the system and the determined risk score and likelihood score, the relevance score comprising one or more clinical tests relevant to confirming or rejecting the possible pathogenic infection of the person, and further comprising a ranking of the clinical tests based on a likelihood of confirming or rejecting the possible pathogenic infection of the person using those tests; and (v) reporting, via a user interface, the determined relevance score.

According to an embodiment, the system further includes: conducting, by a healthcare professional, one or more of the one or more clinical tests identified in the determined relevance score; and diagnosing the patient with one or more of the one or more pathogens or pathogen categories. According to an embodiment, the system further includes treating the patient for the diagnosed one or more pathogens or pathogen categories.

According to an embodiment, the triggering indicator is provided manually. According to an embodiment, the triggering indicator is automatically generated based on medical information about the patient.

According to an embodiment, the relevance score further comprises, for each of the one or more clinical tests, a relevance of the clinical test to a diagnosis of the possible pathogenic infection of the person.

According to an embodiment, the relevance score comprises a first, positive value indicating that a positive result of the clinical test helps confirm diagnosis. According to an embodiment, a larger positive value indicates that the associated clinical test is more relevant to a diagnosis and/or is more commonly ordered in diagnosing the likely infection, and wherein a smaller positive value indicates that the associated clinical test is less relevant to a diagnosis and/or is less commonly ordered in diagnosing the likely infection.

According to an embodiment, the relevance score comprises a second, negative value indicating that a negative result of the clinical test helps rule out other possibilities of diagnosis. According to an embodiment, a larger negative value indicates that the associated clinical test is more relevant to excluding other possibilities of diagnosis, and wherein a smaller negative value indicates that the associated clinical test is less relevant to a diagnosis and/or is less commonly ordered in diagnosing the likely infection.

According to an embodiment, the one or more clinical tests are ranked based on the first, positive value, and wherein the ranking is provided via the user interface.

In various implementations, a processor or controller may be associated with one or more storage media (generically referred to herein as "memory," e.g., volatile and non-volatile computer memory such as RAM, PROM, EPROM, and EEPROM, floppy disks, compact disks, optical disks, magnetic tape, etc.). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects as discussed herein. The terms "program" or "computer program" are used herein in a generic sense to refer to any type of computer code (e.g., software or microcode) that can be employed to program one or more processors or controllers.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

## Brief Description of the Drawings

In the drawings, like reference characters generally refer to the same parts throughout the different views. Also, the drawings are not necessarily to scale, emphasis instead generally being placed upon illustrating the principles of the various embodiments.

FIG. 1 is a flowchart of a method for diagnosing pathogenic infection of a person, as carried out by a system in accordance with an embodiment.

FIG. 2 is a flowchart of a method for diagnosing pathogenic infection of a person, as carried out by a system in accordance with an embodiment.

FIG. 3 is a flowchart of a method for training a risk classifier and a likelihood classifier of ab infection analysis system, as carried out by a system in accordance with an embodiment.

FIG. 4 is a schematic representation of a system for diagnosing pathogenic infection of a person, in accordance with an embodiment.

## Detailed Description of Embodiments

The present disclosure describes various embodiments of a system for characterizing a likelihood of infection of a person upon an early suspicion of infection. More generally, Applicant has recognized and appreciated that it would be beneficial to provide a system configured to diagnose a pathogenic infection of the person. The system receives a trigger comprising data indicative of a possible pathogenic infection of a person, often a patient at a patient care facility. The system uses a risk classifier and medical information about the patient to determine a risk score for the patient comprising a likelihood that one or more body systems is infected. The system also uses a likelihood classifier and medical information about the patient to determine a likelihood score for the patient comprising an identification of one or more pathogens or pathogen categories that could be causing an infection. With the risk score and the likelihood score, the system uses a relevance classifier to determine a relevance score comprising one or more clinical tests most relevant to confirming or rejecting the possible pathogenic infection of the person. According to an embodiment, the healthcare professional receives the information and conducts one or more of the clinical tests identified in the determined relevance score, and based on the output of the tests diagnoses the patient with one or more pathogens or pathogen categories. The healthcare professional then treats the patient for the diagnosed one or more pathogens or pathogen categories.

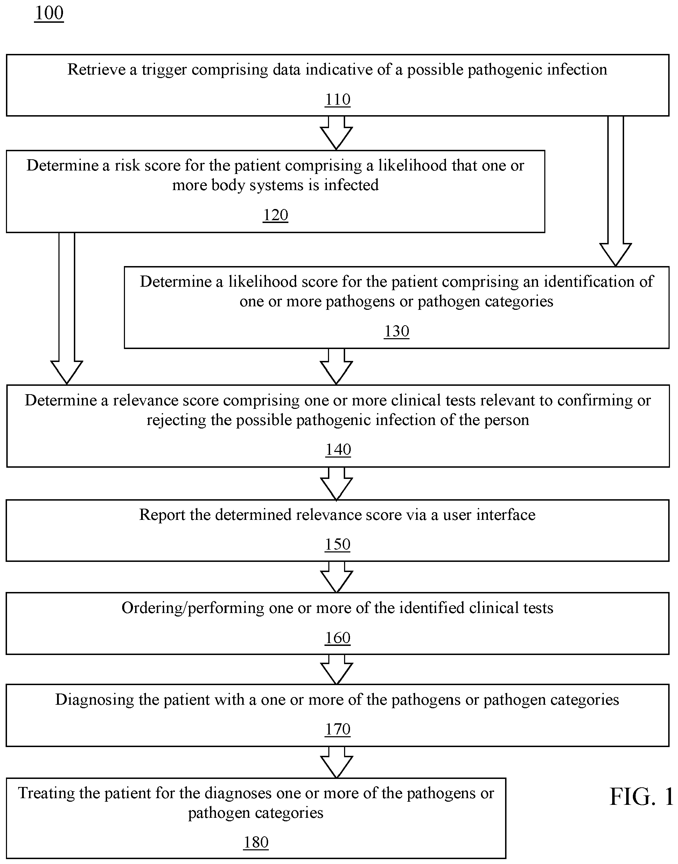

FIG. 1 shows a flowchart of an embodiment of a method 100 for diagnosing pathogenic infection of a person using an infection analysis system. The infection analysis system can be any of the systems described or otherwise envisioned herein, and may comprise any of the components described or otherwise envisioned herein.

At step 110 of the method, the infection analysis system receives a trigger comprising data indicative of a possible pathogenic infection of a person. The trigger may be a manual trigger or an automated trigger. The received data will trigger downstream analysis by the infection analysis system.

A person or patient referred to herein can be any person or patient for whom early diagnosis of infection is desired. For example, it can be a person in a hospital or other healthcare setting that is being monitored, admitted, or otherwise cared for. Since hospital-acquired infections are common and a pressing healthcare concern, patients are regularly monitored for signs of infection. Alternatively, the person or patient may be remote from a healthcare setting but providing information or data to the system, such as through a portal, telemedicine link, or any other connection. Many other people or patients can participate in the system for early infection diagnosis.

According to an embodiment, a trigger may be something performed manually. For example, a manual trigger can be any action taken by a healthcare professional or any other monitoring person. The manual trigger may be made at the onset of infection suspicion, or when clinical data such as ongoing monitoring indicates a possible infection situation. A manual trigger may be activation of the system via a user interface, among many other possible actions. Alternatively, the trigger may be a lack of action. For example, the system may be programmed to perform an analysis for every patient or only certain patients if a certain threshold or state exists. As just one example, the system may perform the analyses described or otherwise envisioned herein for every patient once a fever reaches 38 °C or higher. Accordingly, by failing to deactivate the system and letting the system perform the analysis, the professional is manually activating the system.

According to an embodiment, the trigger may be provided automatically. Thus the system may comprise a defined set of criteria to trigger the system. This can be empirical rules such as elevated temperature (*e.g.*, >38 °C) and/or increased white blood cell count (*e.g.*, WBC > 10,000/mL), which can be enabled by connecting the proposed system to an electronic medical record (EMR) system or a monitoring system where the patient's vital signs and/or lab test results are charted or monitored. Alternatively, clinical criteria such as systemic inflammatory response syndrome (SIRS) criteria can be used to trigger the system, particularly when sepsis is the infection of concern. According to an embodiment, another system may comprise a predictive algorithm that determines a suspicion of infection and sends that prediction to the system as a trigger. Many other triggers are possible. Automated triggers and thresholds for triggers can be defined by a user, a healthcare facility, programming of the system, and/or by other sources.

At step 120 of the method, once a trigger is received by the system, the system uses a risk classifier and medical information about the patient to determine a risk score for the patient comprising a likelihood that one or more body systems is infected. According to an embodiment, the risk score may comprise a likelihood for one or more predefined body systems or predefined body system categories. These systems or categories may be, for example, extracted from clinical guidelines defined by healthcare associations, or they can be specified by the physician or another person who is one of the users or designers or caretakers of the system. For example, according to the National Healthcare Safety Network (NHSN), hospital acquired infection are categorized as infections to one or more of the following body systems: (i) bone and joint; (ii) central nervous system; (iii) cardiovascular system; (iv) eye, ear, nose, throat, or mouth; (v) gastrointestinal system; (vi) respiratory system; (vii) reproductive tract; (viii) skin and soft tissue; and/or (ix) urinary system. Described in greater detail below is the risk classifier used to determine the risk score.

According to an embodiment, the output of the risk score determination, which may be the risk score and which may or may not be provided to a healthcare professional at any stage or point in the method, is a ranking or ranked list of one or more predefined body systems that may or may not be affected by a potential infection. According to an embodiment, each predefined body system is assigned a predicted risk score, with higher value indicating stronger suspicion.

At step 130 of the method, once a trigger is received by the system, the system uses a likelihood classifier and medical information about the patient to determine a likelihood score for the patient comprising an identification of one or more pathogens or pathogen categories that could be causing an infection. Step 130 may be performed before, after, or simultaneously with step 120 of the method. According to an embodiment, the likelihood score comprises a likelihood of one or more predefined pathogen or predefined pathogen categories. These pathogen or pathogen categories may be, for example, defined by healthcare associations, or they can be specified by the physician or another person who is one of the users or designers or caretakers of the system. A predefined category can be defined as general categories of microorganisms such as bacteria, viruses, fungi and protozoa, or it can be defined more specifically, for example breaking down bacteria category into gram-positive versus gram-negative, and/or bacilli versus cocci. A category may also be specified by the healthcare professional, particularly when the professional has a strong suspicion of the microorganism that causes the infection - for example, Methicillin-resistant *Staphylococcus aureus* (MRSA) should be highly suspected when other patients in the same healthcare facility have been diagnosed with MRSA infection and have shown similar symptoms.

According to an embodiment, the output of the likelihood score determination, which may be the likelihood score and which may or may not be provided to a healthcare professional at any stage or point in the method, is a ranking or ranked list of one or more predefined microorganisms or pathogen categories. According to an embodiment, each predefined microorganism is assigned a predicted likelihood score, with higher value indicating stronger suspicion. Described in greater detail below is the likelihood classifier used to determine the likelihood score.

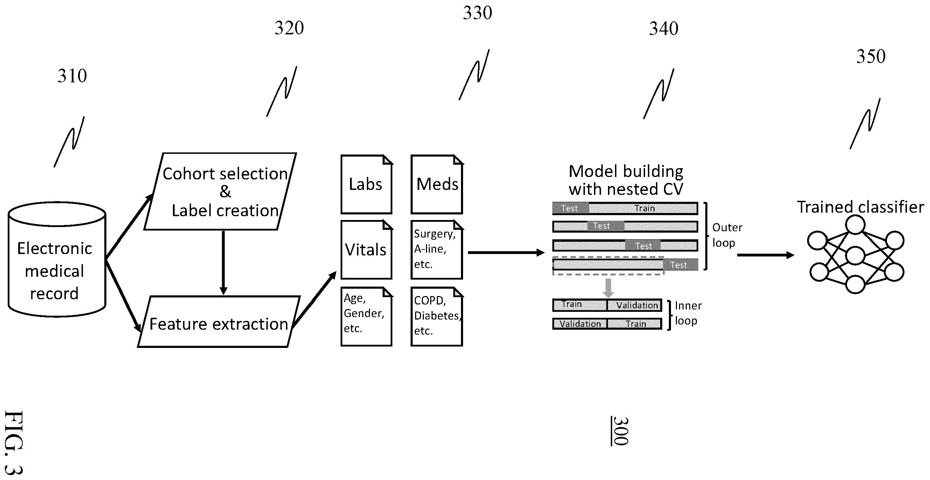

FIG. 3 shows a flowchart of a method 300 for training the risk classifier and the likelihood classifier. According to an embodiment, the system utilizes a retrospective EMR dataset 310 to train the classifiers. Many publicly available datasets can serve this purpose, for example MIMIC-III which is a freely accessible critical care database. Alternatively or additionally, the database may be a private dataset, such as one at the facility using the system, among other datasets.

According to an embodiment, the system leverages multi-modal information such as diagnosis codes, notes and clinical test results to identify infection patients. For example, a patient may be selected when the patient: 1) has at least one diagnosis code (*e.g.*, ICD-9 or ICD-10) indicating infection; or 2) is confirmed to be infected according to a healthcare providers' notes; and/or 3) has clear evidence of infections in clinical test results (*e.g.*, *Streptococcus agalactiae* found in blood indicates a bloodstream infection).

At 320, the system extracts information regarding the confirmed microorganism and infected body system of these patients from the same source data, and matches the confirmed microorganism and infected body system with the respective predefined categories. According to an embodiment, patients that do not have any infected body system and do not have any confirmed microorganisms within the predefined categories will be dropped.

The system may also assign each selected infection patient a timestamp marking the first clinical suspicion of infection. This timestamp can be defined from multiple data sources, such as the first note indicating infection, or the order time of the first microbiology laboratory test, or the prescription time of the first therapeutic antibiotic, among other sources or times. In circumstances where the extracted information conflicts between data sources, a set of rules is applied. Namely, patients with inconsistent microorganisms or body system labels will be dropped, and patients with multiple suspected infection onset times for the same infection will be assigned the earliest time. After all information is integrated, the system creates a final list or table or other data structure with each remaining infection patient having a: 1) corresponding ID; 2) infected body system label; 3) invading microorganism label; and 4) suspected infection onset time in reference to their admission, among other possible information.

At 330, once an infection cohort and associated labels is created, the system extracts clinical data available before (and optionally during and/or after) the suspected infection onset for each patient. These clinical data can include, among many other things: 1) vitals such as heart rate and temperature; 2) lab results such as white blood cell count and blood lactate level; 3) medications such as prophylactic antibiotics and corticosteroids; 4) interventions such as surgery and the placement of an arterial line; 5) comorbidities such as diabetes and hypertension; and/or demographics such as age and gender.

According to an embodiment, there may be vitals and labs that may have multiple measurements. Accordingly, the system may define an observation window, such as 24 hours, 48 hours, or some other timeframe, that precedes the suspected infection onset, and may then generate summary statistics from the labs and vitals that occurred within the window. For example, for medications, interventions, and comorbidities, the system can convert them into binary labels to mark whether the patient has a given comorbidity, and whether the patient has received a given intervention/medication before infection suspicion. The system can also apply mean imputation for the missing values. Through these processes the system represents each patient with a feature vector, where each feature is either a summary statistic of a clinical measurement or is describing the patient's past medication/intervention history, comorbidities, or demographics.

The system then uses these feature vectors to independently train the risk classifier with infected body system labels as the target of prediction for the former, and the likelihood classifier with invading microorganism labels as the target prediction. The system may assume independence of infected body systems, and independence of invading microorganisms, such that the multi-label classification problem can be transformed to multiple binary classification problems. Classical approaches of binary predictions can then be used to obtain the risk score and the likelihood score.

According to an embodiment, at 340 the system is trained via one or more of the following steps, using predicting microorganisms as an example, although many other methods and training steps or systems may be utilized. As an initial step A, the system selects a set of machine learning algorithms to use to train and compare. Examples of candidate algorithms are logistic regression, support vector machines, random forest, and many others. At step B, for each candidate model, a binary classifier is trained for each predefined microorganism category, and overall performance is calculated across categories. For each predefined category of microorganism, a binary label is generated indicating if the patient is infected by the given category of microorganism or not. The system uses nested cross validation techniques to train the binary classifier where the inner loop is used for hyperparameter tuning and the outer loop is used for fitting the model. A performance score of choice is defined, such as under the ROC curve, and the average across all outer folds is taken as the performance score of predicting the given microorganism category. These sub-steps in step B are repeated to obtain performance scores for all the microorganism categories and to compute the average performance for each candidate model. All of B is then repeated for all the candidate models. At step C (350), the machine learning model with the best overall performance for all the microorganism categories is selected, and this trained model will be used to generate likelihood scores of microorganisms for a newly suspected infection patient.

Once the system has a risk score comprising one or more predefined body systems and the risk of each being infected, and has a likelihood score comprising one or more predefined microorganisms or categories measuring the likelihood of some or all of them being the cause of the possible infection, the system can calculate a relevance score comprising one or more predefined clinical tests measuring the relevance of each to infection diagnosis.

Accordingly, at step 140 of the method, the system uses a relevance classifier, and the determined risk score and determined likelihood score, to determine a relevance score comprising one or more clinical tests relevant for confirming or rejecting the possible pathogenic infection of the person. The relevance score also comprises a ranking of the clinical tests based on a likelihood of confirming or rejecting the possible pathogenic infection of the person using those tests. Step 140 is performed after steps 120 and 130, as the relevance score is determined using the determined risk score and determined likelihood score.

According to an embodiment, the relevance score indicates which predefined clinical test is more relevant to the diagnosis of infection given the prediction of the infection. This is based on the risk score of the infected body system and the likelihood score of the pathogen. Here are just a few examples of clinical tests that may be predefined, although the system may utilize any clinical test for this step: chest x-ray, echocardiogram, electrocardiography, spinal tap, basic metabolic panel, electrolyte panel, complete blood count, arterial blood gas, urinalysis, sputum culture test, nasal swab test, chest computed tomography (CT) scan, blood culture test, and/or stool culture test, among many others.

According to an embodiment, upon determination of the risk score and the likelihood score, the system by default triggers the relevance classifier to identify one or more relevant clinical tests for diagnosing the infection of the highest suspicion, that is, the infection caused by the microorganism with the highest likelihood score and that affects the body system with the highest risk score. This default behavior can be turned off, such as in the situation when the physician believes there is already has enough information from the predicted microorganism and the infected body system to make decisions for clinical tests. Alternatively, the system can be configured such that a weighted sum of relevance scores for clinical tests is produced. For example, the relevance scores for the top three suspected infection can be weighted as 3, 2, and 1 respectively. This configuration is useful when more than one invading microorganism and infected body system are highly suspected. The relevance classifier can be used in a standalone mode where the healthcare professional or other use manually inputs ranks and/or weights of suspected body systems and microorganisms. This standalone mode is useful if a good estimation of infection is already established through other means.

According to an embodiment, the output of the relevance classifier is a list of one or more predefined clinical tests, each assigned with a pair of scores. The first score is a positive value indicating that a positive result of the clinical test helps confirm diagnosis, and the second score is a negative value indicating that a negative result of the clinical test helps rule out other possibilities. A higher absolute value indicates that the clinical test is more relevant and is more commonly ordered in diagnosing the infection of interest, where a value close to zero indicates the clinical test is not relevant.

According to an embodiment, the list of clinical tests can be presented to the end user in several different ways. One approach is a ranked list in descending order, sorted by the value of the first relevance score of the pair, such that the clinical tests in the beginning of the list are the most relevant tests for confirming diagnosis and are strongly recommended. Another approach is a ranked list in ascending order, sorted by the value of the second relevance score of the pair, such that the clinical tests in the beginning of the list are the most common tests used to rule out illnesses that share similar symptoms or cause similar physiological change of the infection of interest. While the first ranked list may be primarily used by a doctor to guide decision making of ordering clinical tests, the second ranked list provides important information that is easy to neglect, and might be utilized when there are confounding diseases.

The relevance classifier can be trained in many different ways. Described below is just one possible method to train the relevance classifier, although many other training methods may be utilized. At an initial step, records of clinical tests that are ordered between the onset of infection suspicion and the time of infection diagnosis for each infection patient are extracted. The suspected infection onset can be defined as described herein. The time of infection diagnosis can be defined as the time when the infection diagnosis code was first charted in the database, or the time when written notes first indicate the diagnosis of infection, whichever comes earlier. Extracted clinical tests are matched with the predefined set of $k$ categories of clinical tests, such that each infection patient $x$ is represented by a vector of clinical tests $x = \langle t_1, \cdots, t_k \rangle$. Each clinical test t is assigned with one of the three integer values $t \in \{1, -1, 0\}$, where 1 denotes positive result, -1 denotes negative result, and 0 denotes that the clinical test was not ordered for the given patient. A positive result indicates the clinical test found evidence of a particular infection type, and a negative result means otherwise. In a pneumonia example, signs of infiltrate, or consolidation, or cavitation of the lung is considered as a positive result of chest x-ray, whereas an ECG showing a normal reading is a negative result.

The system uses these vectors of clinical tests as the input data to train the model. The targeted output of the model is the paired set of infected body system and the invading microorganism diagnosed for the infection patient denoted as $I = \langle B_i, P_i \rangle$, where $I$ denotes information regarding the infection of patient $x$, $B_i$ and $P_i$ denotes respectively the infected body system and invading microorganism of patient $x$. These labels are created when the risk classifier and the likelihood classifier are trained. The paired set are used as the prediction target such that the multi-label classification problem is converted to a multiclass problem where the dependence between infected body system and invading microorganism is preserved when constructing the model.

According to an embodiment the system may use a decision tree as a base model, although many other approaches are possible. The system may modify the splitting rule of the decision tree model to best suit the current application. This may be because the structure of a decision tree can resemble the current practice of ordering clinical tests, where a series of clinical tests are ordered and each provide further information to help diagnosis either by directly increasing the confidence of diagnosis or by ruling out other possibilities. Thus the splitting rule of the decision tree is modified such that the features that were used in previous nodes are excluded from the selection of features for the current split, as each clinical test typically only needs to be ordered once. Thus, an individual diagnosis decision can be modelled as a decision path of a decision tree, composed of a chain of decisions, each made after the receiving of result from the previous clinical test. In mathematical form, this can be expressed as:

$$f(x) = C_{bias} + \sum_{k=1}^{K} C(x, k) \qquad\qquad (\text{Eq. 1})$$

where $f(x)$ is the prediction function of a tree to diagnose patient $x$, $C(x, k)$ is the contribution of clinical test $k$ to diagnose patient $x$, and $C_{bias}$ is the bias term which is a constant for patient $x$.

The system can then use a probabilistic approach to evaluate, along a single decision path, the contribution of each clinical test in diagnosing the infection, namely the term $C(x, k)$. This can be measured as the change of predicted probabilities after a particular decision is made based on the result of a clinical test from the trained tree.

According to an embodiment, the system may utilize the ensemble method Random Forest to account for the heterogeneity of clinical tests ordered for patients with the same infection type. The system can calculate the contribution of each clinical test $C(x, k)$ for each patient in each tree and aggregate the contributions across trees to compute relevance scores. First, positive and negative results are separated such that the system produces a pair of relevance scores for each clinical test. The pair of scores can be denoted as:

$$R(k, I) = \langle R_+(k, I), R_-(k, I) \rangle \qquad\qquad (\text{Eq. 2})$$

where $R(k, l)$ is the pair of relevance scores of clinical test $k$ in diagnosing infection type $l = \langle B_i, P_i \rangle$. The first score $R_+(k, l)$ measures the average contribution of a positive result in helping to confirm a diagnosis, and the second score $R_-(k, l)$ measures the average contribution of a negative result in ruling out other disease possibilities.

According to an embodiment, the system factors in the confidence of prediction when aggregating the contributions. Contributions of clinical tests that are used in trees which produce the correct prediction are weighted as 1, whereas those used in trees which produce wrong predictions are weighted as 0. A weighted average is then calculated using these weights, which are formulated as

$$R_+(k, l) = \frac{1}{m} \sum_{x \in M} \left( \frac{1}{s} \sum_{j \in S_x} C_j(x, k) \right) \qquad \text{(Eq. 3)}$$

and

$$R_-(k, l) = -\frac{1}{n} \sum_{x \in N} \left( \frac{1}{s} \sum_{j \in S_x} C_j(x, k) \right) \qquad \text{(Eq. 4)}$$

where $M$ is the set of patients of infection type $l$ that have positive results of clinical test $k$, number of patients in the set equals to $m$; $N$ is the set of patients of infection type $l$ that have a negative results of clinical test $k$, number of patients in the set equals to n; s is the total number of trees in the forest which is a constant; $S_x$ is the set of trees in the forest which produces correct prediction for patient $x$; and $C_j(x, k)$ is the contribution of the $k$-th clinical test in $j$-th tree for patient $x$.

According to one embodiment, the intended users of the proposed system can be healthcare providers or other users in one or more of categories. For example, the healthcare providers or other users may fall within one or more of these three categories: physicians, nurses, and healthcare administrators.

According to one embodiment, the system provides two user endpoints, tailored for specific user needs. The first user endpoint is the predicted information of infection, provided in the format of risk scores of infected body systems and likelihood scores of invading microorganisms. The users may initiate one or more of the following actions based on these scores:

1. Physicians may start empirical treatment such as antimicrobial therapy for a strongly predicted bacterial infection;
2. Nurses may increase the monitoring frequency for patients who are predicted to have higher-acuity infections; and/or
3. Healthcare administrators may evoke transmission prevention protocol for predicted infections that are highly contagious.

According to an embodiment, the second user endpoint may be the relevance score of clinical tests. The intended user for this endpoint might be the physician, who may choose to use this information to guide the decision making process for of ordering one or more clinical tests.

At step 150 of the method, the system reports the determined relevance score via a user interface of the system. The user interface may be located with one or more other components of the system, or may located remote from the system and in communication via a wired and/or wireless communications network. Reporting may comprise any report that conveys or communicates the relevance score information. In addition to the relevance score, the report may comprise the risk score and the likelihood score, with their respective rankings. The output may be a ranked table, ranked list, heat map, graph, text, and/or any other format. For example, the output may be provided on a screen, monitor, or other display, and/or may be reported via a textual report, among many other methods.

The relevance score report may comprise the determined ranked list of one or more clinical tests used to diagnose the likely pathogen in the likely body system. The report may also comprise the pair of scores, the first score being a positive value indicating that a positive result of the clinical test helps confirm diagnosis, and the second score being a negative value indicating that a negative result of the clinical test helps rule out other possibilities.

At step 160 of the method, a healthcare professional orders, enacts, performs, or otherwise results in performance of at least one of the highly-ranked clinical tests identified in the relevance score. Since the system has identified a likely pathogen and a likely body system, they system can identify the most relevant clinical tests. Human decision making can be influenced by many factors, and can be very seriously impacted by confirmation bias, leading to poor decisions or biased decisions. In contrast, the system is not subject to confirmation bias, instead utilizing classifiers trained on enormous datasets that no human mind could even begin to experience or process. Among many other types of testing used to diagnosis infection, the at least one highly-ranked clinical test identified in the relevance score and ordered by the healthcare professional may comprise one or more of the following: chest x-ray, echocardiogram, electrocardiography, spinal tap, basic metabolic panel, electrolyte panel, complete blood count, arterial blood gas, urinalysis, sputum culture test, nasal swab test, chest computed tomography (CT) scan, blood culture test, and/or stool culture test, among many others.

According to an embodiment, the selected one or more highly-ranked tests are selected because they are so highly-ranked in the relevance score. Thus, there is a direct application of the relevance score determination in the steps of the method. Indeed, according to an embodiment, the system may be configured to automatically order one or more of the most highly-ranked tests in the relevance score.

At step 170 of the method, the patient is diagnosed with one or more of the highly-ranked pathogens or pathogen categories identified in the likelihood score, affecting one or more of the highly-ranked body systems identified in the risk score. The diagnosis is directly dependent upon the results of the clinical tests ordered on the basis of being highly-ranked in the relevance score. For example, the healthcare professional orders one or more of the highly-ranked clinical tests identified and ranked in the relevance score, receives the results of the one or more tests, and interprets the results to identify one or more of those highly-ranked pathogens or pathogen categories as the pathogen causing the infection.

At step 180 of the method, the patient is treated for infection caused by the one or more pathogens or pathogen categories identified by the system, based on the outcome of the one or more of the highly-ranked clinical tests identified by the relevance score. The infection treatment is based on the pathogen identified by the clinical tests, and may include a wide variety of different treatments including antibiotics and many other treatments.

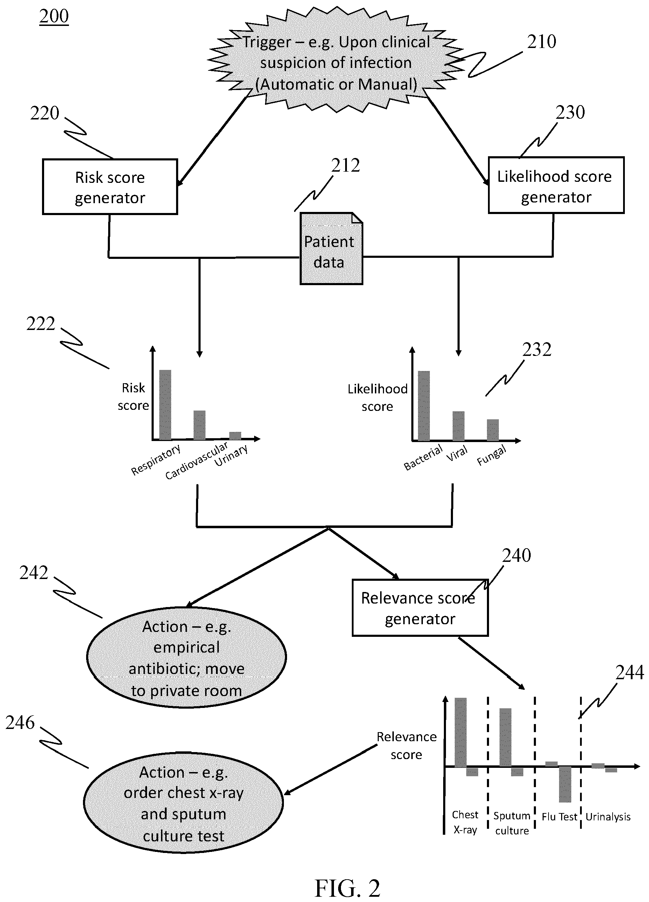

FIG. 2 shows a similar flowchart of a method 200 for diagnosing pathogenic infection of a person using an infection analysis system. The infection analysis system can be any of the systems described or otherwise envisioned herein, and may comprise any of the components described or otherwise envisioned herein.

At 210, a manual or automatic trigger triggers the system to determine a risk score using a risk score generator or classifier at 220 and to determine a likelihood score using a likelihood score generator or classifier at 230. Both the risk score generator or classifier and the likelihood score generator or classifier utilize patient data 212, such as from a medical records database or other source, to generate the respective score.

In both cases the classifiers provide a ranked listing. The risk score generator or classifier comprises a ranked listing of body systems 222. As shown in FIG. 2, in just one embodiment the ranked listing may be a graph of likelihood, percentages, likelihood ratios, or other indication of ranking. In the image, the respiratory, cardiovascular, and urinary body systems are identified with the respiratory system being the most likely.

The likelihood score generator or classifier comprises a ranked listing of pathogen or pathogen categories 232. As shown in FIG. 2, in just one embodiment the ranked listing may be a graph of likelihood, percentages, likelihood ratios, or other indication of ranking. In the image, bacterial, viral, and fungal infections are identified with a bacterial infection being the most likely.

At 242, the ranked listing of body systems 222 and the ranked listing of pathogen or pathogen categories 232 may comprise sufficient information to begin a treatment regime, such as a medicine like antibiotic, moving the patient to a private room, and/or other treatments.

At 240, a relevance classifier uses the determined risk score and determined likelihood score, to determine a relevance score comprising one or more clinical tests relevant to confirming or rejecting the possible pathogenic infection of the person. The relevance score also comprises a ranking of the clinical tests based on a likelihood of confirming or rejecting the possible pathogenic infection of the person using those tests.

An example of a relevance score 244 is shown in FIG. 2. The relevance score 244 comprises a series of ranked clinical tests including a chest x-ray, sputum culture, flu test, and urinalysis. Each clinical test is also associated with two indicators, such as numbers, shown as bars in the graph in FIG. 2. For the chest x-ray for example, there is a tall positive bar comprising an indication that a positive finding of the test - in other words an indication of infection based on the result of the test - is strong evidence for diagnosis of the identified infection. There is also a much shorter negative bar comprising an indication that a negative finding of the test - in other words an indication of no infection based on the result of the test - is not very good evidence to weigh against a diagnosis of the identified infection. In contrast, the flu test is just the opposite.

In the embodiment shown in FIG. 2, the four tests are ranked and displayed in an order based on the first relevance score of the pair of scores, such that the clinical tests in the beginning of the list are the most relevant tests for confirming diagnosis. Many other rankings are possible.

Based on the results of the relevance score, the system indicates actions to take and a healthcare professional carries out those actions at 246. Based on the outcomes of the tests and the clinical test diagnostic values provided via the relevance score, a diagnosis of the likely pathogen or pathogen category is made and the patient can be treated for the diagnosed pathogen or pathogen category.

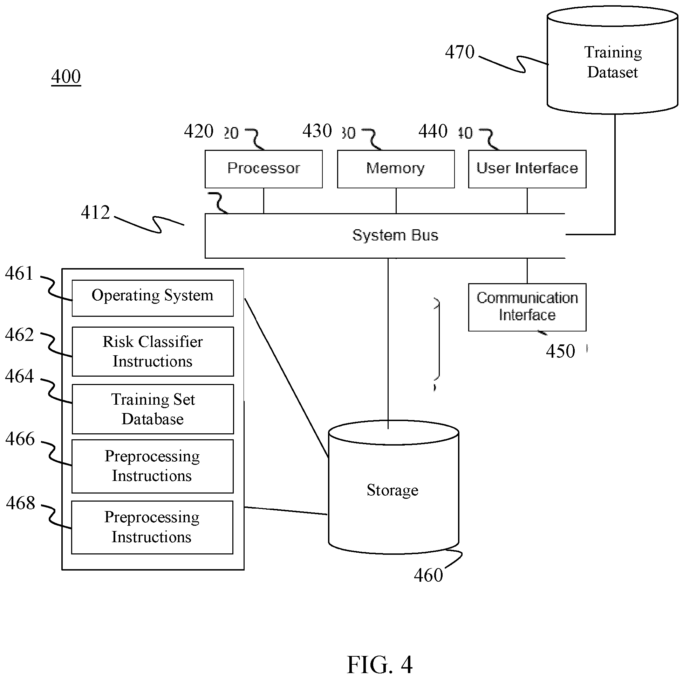

FIG. 4 shows an infection analysis system 400 for diagnosing pathogenic infection of a person using an infection analysis system. The infection analysis system can be any of the systems described or otherwise envisioned herein, and may comprise any of the components described or otherwise envisioned herein.

According to an embodiment, system 400 comprises one or more of a processor 420, memory 430, user interface 440, communications interface 450, and storage 460, interconnected via one or more system buses 412. It will be understood that FIG. 4 constitutes, in some respects, an abstraction and that the actual organization of the components of the system 400 may be different and more complex than illustrated.

According to an embodiment, system 400 comprises a processor 420 capable of executing instructions stored in memory 430 or storage 460 or otherwise processing data to, for example, perform one or more steps of the method. Processor 420 may be formed of one or multiple modules. Processor 420 may take any suitable form, including but not limited to a microprocessor, microcontroller, multiple microcontrollers, circuitry, field programmable gate array (FPGA), application-specific integrated circuit (ASIC), a single processor, or plural processors.

Memory 430 can take any suitable form, including a non-volatile memory and/or RAM. The memory 430 may include various memories such as, for example L1, L2, or L3 cache or system memory. As such, the memory 430 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 400. It will be apparent that, in embodiments where the processor implements one or more of the functions described herein in hardware, the software described as corresponding to such functionality in other embodiments may be omitted.

User interface 440 may include one or more devices for enabling communication with a user. The user interface can be any device or system that allows information to be conveyed and/or received, and may include a display, a mouse, and/or a keyboard for receiving user commands. In some embodiments, user interface 440 may include a command line interface or graphical user interface that may be presented to a remote terminal via communication interface 450. The user interface may be located with one or more other components of the system, or may located remote from the system and in communication via a wired and/or wireless communications network.

Communication interface 450 may include one or more devices for enabling communication with other hardware devices. For example, communication interface 450 may include a network interface card (NIC) configured to communicate according to the Ethernet protocol. Additionally, communication interface 450 may implement a TCP/IP stack for communication according to the TCP/IP protocols. Various alternative or additional hardware or configurations for communication interface 450 will be apparent.

Storage 460 may include one or more machine-readable storage media such as read-only memory (ROM), random-access memory (RAM), magnetic disk storage media, optical storage media, flash-memory devices, or similar storage media. In various embodiments, storage 460 may store instructions for execution by processor 420 or data upon which processor 420 may operate. For example, storage 460 may store an operating system 461 for controlling various operations of system 400.

It will be apparent that various information described as stored in storage 460 may be additionally or alternatively stored in memory 430. In this respect, memory 430 may also be considered to constitute a storage device and storage 460 may be considered a memory. Various other arrangements will be apparent. Further, memory 430 and storage 460 may both be considered to be non-transitory machine-readable media. As used herein, the term non-transitory will be understood to exclude transitory signals but to include all forms of storage, including both volatile and non-volatile memories.

While infection analysis system 400 is shown as including one of each described component, the various components may be duplicated in various embodiments. For example, processor 420 may include multiple microprocessors that are configured to independently execute the methods described herein or are configured to perform steps or subroutines of the methods described herein such that the multiple processors cooperate to achieve the functionality described herein. Further, where one or more components of system 400 is implemented in a cloud computing system, the various hardware components may belong to separate physical systems. For example, processor 420 may include a first processor in a first server and a second processor in a second server. Many other variations and configurations are possible.

According to an embodiment, system 400 comprises or is in communication with a database 470 such as one or more training datasets for training one or more of the risk classifier, likelihood classifier, and/or the relevance classifier. The system utilizes a retrospective EMR dataset to train the classifiers. Many publicly available datasets can serve this purpose, for example MIMIC-III, which is a freely accessible critical care database. Alternatively or additionally, the database may be a private dataset, such as one at the facility using the system, among other datasets. According to an embodiment the relevance classifier can be trained using an EMR dataset comprising records of clinical tests that are ordered between the onset of infection suspicion and the time of infection diagnosis for each infection patient are extracted. Other datasets and information are possible.

According to an embodiment, storage 460 of infection analysis system 400 may store one or more algorithms and/or instructions to carry out one or more functions or steps of the methods described or otherwise envisioned herein. For example, processor 420 may comprise, among other instructions, risk classifier instructions 462, likelihood classifier instructions 464, relevance classifier instructions 466, user interface instructions 468, and/or other instructions.

According to an embodiment, risk classifier instructions 462 direct the system to determine a risk score for the patient comprising a likelihood that one or more body systems is infected. To do this, the risk classifier instructions may direct the system to use a risk classifier of the system, and medical records for the patient such as those obtained from an electronic records system and otherwise obtained by or provided to the system in the analysis. According to an embodiment, the risk score may comprise a likelihood for one or more predefined body systems or predefined body system categories. These systems or categories may be, for example, extracted from clinical guidelines defined by healthcare associations, or they can be specified by the physician or another person who is one of the users or designers or caretakers of the system. Accordingly, a system database may comprise a listing of the extracted or identified body systems or body system categories. The risk classifier instructions 462 may also direct the system to train the risk classifier with training data as described or otherwise envisioned herein.

According to an embodiment, likelihood classifier instructions 464 direct the system to generate a risk score for the patient comprising an identification of one or more pathogens or pathogen categories that could be causing an infection. To do this, the likelihood classifier instructions may direct the system to use a likelihood classifier of the system, and medical records for the patient such as those obtained from an electronic records system and otherwise obtained by or provided to the system in the analysis. According to an embodiment, the likelihood score comprises a likelihood of one or more predefined pathogen or predefined pathogen categories. These pathogen or pathogen categories may be, for example, defined by healthcare associations, or they can be specified by the physician or another person who is one of the users or designers or caretakers of the system. A predefined category can be defined as general categories of microorganisms such as bacteria, viruses, fungi and protozoa, or it can be defined more specifically, for example breaking down bacteria category into gram-positive versus gram-negative, and/or bacilli versus cocci. Accordingly, a system database may comprise a listing of the extracted or identified pathogens and pathogen categories. The likelihood classifier instructions 464 may also direct the system to train the likelihood classifier with training data as described or otherwise envisioned herein.

According to an embodiment, relevance classifier instructions 466 direct the system to determine a relevance score comprising one or more clinical tests relevant to confirming or rejecting the possible pathogenic infection of the person. To do this, the relevance classifier instructions may direct the system to use the determined risk score and determined likelihood score. The relevance score comprises a ranking of the clinical tests based on a likelihood of confirming or rejecting the possible pathogenic infection of the person using those tests. According to an embodiment, the relevance score indicates which predefined clinical test is more relevant to the diagnosis of infection given the prediction of the infection. This is based on the risk score of the infected body system and the likelihood score of the pathogen.

According to an embodiment, user interface instructions 468 direct the system to receive information from and/or provide information to a user via user interface 540. For example, the user interface instructions 468 may be used to receive information about a patient, and/or to provide information to a healthcare professional including the output of the relevance score, and including but not limited to the example outputs shown, described, or otherwise discussed herein.

The system and methods described and otherwise envisioned herein provide numerous advantages over prior art systems, including earlier and faster diagnosis of infection. This is particularly important, and potentially lifesaving, when the infection is a hospital-acquired infection which can be difficult to treat. According to an embodiment, the system can be integrated with inpatient EMR system or monitor system where it provides assisted intelligence in diagnosing hospital acquired infections. The system allows easy configuration of predefined infection categories, therefore one can modify it to predict more specialized infection types such as procedure and/or device-associated infections, or infections caused by specific bacterial strains. The system can also be expanded to generate relevance scores of interventions and medications for treating the suspected infection, provided a set of predefined interventions/medications is available. Finally, the risk score and likelihood score generator can be adapted to use in an outpatient setting when patients' physiological data is measured through health tracking devices.

Among many other advantages, the systems and methods provide the following: 1) a hierarchical framework with multiple user endpoints and customizable trigger conditions that automate clinical workflow; 2) large-volume, multi-modal clinical data used to train a machine learning model that is tailored to mimic the clinical decision making process of infection diagnosis; and 3) individual recommendations of clinical tests for infection diagnosis, with information on how the result of a given clinical test influences the diagnosis. The proposed system can be composed of at least three multi-label classifiers: 1) a risk score generator to score infected body system, 2) a likelihood score generator to score invading microorganism, and 3) a relevance score generator to score clinical test for infection diagnosis. The three classifiers are trained independently from retrospective clinical data; but are configured to be triggered in a hierarchal order, where the output of the first two classifiers are the input of the third classifier. This hierarchical nature is an important and advantageous feature of the system. Additionally, there are no other clinical decision support systems that enable assisted infection diagnosis with such enriched information and such degree of automation.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "having," "composed of," and the like are to be understood to be openended, i.e., to mean including but not limited to.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

**Claims**

1. A system (400) for diagnosing pathogenic infection of a person, the system comprising:

a processor (420) configured to: (i) receive a trigger comprising data indicative of a possible pathogenic infection of the person; (ii) determine, using a risk classifier and medical information about the patient, a risk score for the patient comprising a likelihood that one or more body systems is infected; (iii) determine, using a likelihood classifier and the medical information, a likelihood score for the patient comprising an identification of one or more pathogens or pathogen categories that could be causing an infection; (iv) determine a relevance score using a relevance classifier and the determined risk score and likelihood score, the relevance score comprising one or more clinical tests relevant to confirming or rejecting the possible pathogenic infection of the person, and further comprising a ranking of the clinical tests based on a likelihood of confirming or rejecting the possible pathogenic infection of the person using those tests; and
a user interface (450) configured to report the determined relevance score.

2. The system of claim 1, wherein the relevance score provided via the user input further comprises, for each of the one or more clinical tests, a relevance of the clinical test to a diagnosis of the possible pathogenic infection of the person.

3. The system of claim 1, wherein the relevance score provided via the user input further comprises a first, positive value indicating that a positive result of the clinical test helps confirm diagnosis.

4. The system of claim 3, wherein a larger positive value indicates that the associated clinical test is more relevant to a diagnosis and/or is more commonly ordered in diagnosing the likely infection, and wherein a smaller positive value indicates that the associated clinical test is less relevant to a diagnosis and/or is less commonly ordered in diagnosing the likely infection.

5. The system of claim 3, wherein the one or more clinical tests are ranked based on the first, positive value, and wherein the ranking is provided via the user interface.

6. The system of claim 3, wherein the relevance score comprises a second, negative value indicating that a negative result of the clinical test helps rule out one or more other diagnostic possibilities.

100

Retrieve a trigger comprising data indicative of a possible pathogenic infection

110

Determine a risk score for the patient comprising a likelihood that one or more body systems is infected

120

Determine a likelihood score for the patient comprising an identification of one or more pathogens or pathogen categories

130

Determine a relevance score comprising one or more clinical tests relevant to confirming or rejecting the possible pathogenic infection of the person

140

Report the determined relevance score via a user interface

150

Ordering/performing one or more of the identified clinical tests

160

Diagnosing the patient with a one or more of the pathogens or pathogen categories

170

Treating the patient for the diagnoses one or more of the pathogens or pathogen categories

180

FIG. 1

200

FIG. 2

FIG. 3

400

420 20  430 30  440 40

470 ⟿ Training Dataset

| Processor | Memory | User Interface |

412 ⟿

System Bus

461 ⟿ Operating System

462 ⟿ Risk Classifier Instructions

464 ⟿ Training Set Database

466 ⟿ Preprocessing Instructions

468 ⟿ Preprocessing Instructions

Communication Interface

450

Storage

460

FIG. 4

Europäisches
Patentamt

European
Patent Office

Office européen
des brevets

**EUROPEAN SEARCH REPORT**

Application Number

EP 20 15 0274

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/144943 A1 (KHATRI PURVESH [US] ET AL) 16 May 2019 (2019-05-16) * The whole document, in particular: Paragraphs [0007], [0008], [0032], [0034], [0135], [0237], [0243], [0248]; Figure 3A. * | 1-6 | INV. G16H50/20 G16H50/30 |
| X | US 2018/246313 A1 (ESHEL YOCHAY SHLOMO [IL] ET AL) 30 August 2018 (2018-08-30) * The whole document, in particular: Paragraphs [0006], [0007], [0040], [0041], [0165], [0166]; Figure 2. * | 1-6 | |
| X | US 2002/107641 A1 (SCHAEFFER ANTHONY J [US] ET AL) 8 August 2002 (2002-08-08) * The whole document, in particular: Paragraphs [0018] - [0032], [0044] - [0048], [0052], [0060], [0061], [0077] - [0083]. * | 1-6 | |
| X | YING-JUI CHANG ET AL: "Predicting Hospital-Acquired Infections by Scoring System with Simple Parameters", PLOS ONE, vol. 6, no. 8, 24 August 2011 (2011-08-24) , page e23137, XP055708707, DOI: 10.1371/journal.pone.0023137 * The whole document, in particular: Sections "Abstract", "Introduction", "Methods". * | 1-6 | TECHNICAL FIELDS SEARCHED (IPC) G16H |

-/--

The present search report has been drawn up for all claims

| Place of search | Date of completion of the search | Examiner |
|---|---|---|
| Munich | 25 June 2020 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CLAUDIA EHRENTRAUT ET AL: "Detecting hospital-acquired infections: A document classification approach using support vector machines and gradient tree boosting", HEALTH INFORMATICS JOURNAL, vol. 24, no. 1, 1 March 2018 (2018-03-01), pages 24-42, XP055708710, GB ISSN: 1460-4582, DOI: 10.1177/1460458216656471 * The whole document, in particular: Sections "Abstract", "Introduction", "Method". * | 1-6 | |

----

TECHNICAL FIELDS
SEARCHED      (IPC)

The present search report has been drawn up for all claims

| Place of search | Date of completion of the search | Examiner |
|---|---|---|
| Munich | 25 June 2020 | Nagele, Stefan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 20 15 0274

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2020

| Patent document cited in search report | | Publication date | Patent family member(s) | | Publication date |
|---|---|---|---|---|---|
| US 2019144943 | A1 | 16-05-2019 | AU 2017278254 A1 | | 15-11-2018 |
| | | | BR 112018075288 A2 | | 19-03-2019 |
| | | | CA 3022616 A1 | | 14-12-2017 |
| | | | CN 109312411 A | | 05-02-2019 |
| | | | EP 3464645 A1 | | 10-04-2019 |
| | | | JP 2019520042 A | | 18-07-2019 |
| | | | KR 20190015294 A | | 13-02-2019 |
| | | | US 2019144943 A1 | | 16-05-2019 |
| | | | WO 2017214061 A1 | | 14-12-2017 |
| US 2018246313 | A1 | 30-08-2018 | AU 2016322966 A1 | | 19-04-2018 |
| | | | CA 2998829 A1 | | 23-03-2017 |
| | | | CN 108474934 A | | 31-08-2018 |
| | | | EP 3350644 A1 | | 25-07-2018 |
| | | | JP 2018534605 A | | 22-11-2018 |
| | | | US 2018246313 A1 | | 30-08-2018 |
| | | | US 2020049970 A1 | | 13-02-2020 |
| | | | WO 2017046799 A1 | | 23-03-2017 |
| US 2002107641 | A1 | 08-08-2002 | US 2002107641 A1 | | 08-08-2002 |
| | | | US 2008319800 A1 | | 25-12-2008 |

EPO FORM P0459

For more details about this annex : see Official Journal of the European Patent Office, No. 12/82